# EUROPÄISCHE PATENTSCHRIFT

(11) **EP 2 168 974 B1**
(45) Veröffentlichungstag und Bekanntmachung des Hinweises auf die Patenterteilung: **02.07.2014**
(21) Anmeldenummer: 09100011.7
(22) Anmeldetag: 11.08.1997
(51) Int. Cl.: C07J 53/00

(54) **Verfahren zur Herstellung von Drospirenon (6ß, 7ß; 15ß,16ß-Dimethylene-3-oxo-17alpha-pregn-4-ene-21,17-carbolactone, DRSP)**
Process for the preparation of Drospirenon (6ß, 7ß; 15ß,16ß-Dimethylene-3-oxo-17alpha-pregn-4-ene-21,17-carbolactone, DRSP)
Procédé de preparation de Drospirenon (6ß, 7ß; 15ß,16ß-diméthylène-3-oxo-17alpha-pregn-4-ene-21,17-carbolactone, DRSP)

(30) Priorität: 12.08.1996 DE 19633685
(43) Veröffentlichungstag der Anmeldung: 31.03.2010
(62) Teilanmeldung aus: 01250277.9
(73) Patentinhaber: Bayer Intellectual Property GmbH, 40789 Monheim am Rhein (DE)
(72) Erfinder: Mohr, Jörg-Thorsten, 59423, Unna (DE); Nickisch, Klaus, 12307, Berlin (DE)
(74) Vertreter: BIP Patents

(56) Entgegenhaltungen:
- EP-A- 0 051 143
- EP-A- 0 075 189
- WO-A-90/14344
- D. BITTLER ET AL: "Synthesis of Spirorenone - A Novel Highly Active Aldosterone Antagonist", ANGEWANDTE CHEMIE. INTERNATIONAL EDITION., Bd. 21, Nr. 9, 1982, Seiten 696-697, XP002047531, WEINHEIM DE
- A. PLATTNER ET AL: "Über Steroide und Sexualhormone. 155. Über 3.alpha,5-dioxy-koprostan und zwei epimere 3,4-Dioxy-cholestane", HELVETICA CHIMICA ACTA., Bd. 31, Nr. 6, 1948, Seiten 1822-1831, XP002690735, CHVERLAG HELVETICA CHIMICA ACTA. BASEL. ISSN: 0018-019X
- B. JANSEN ET AL: "THE CONVERSION OF (-)- AND (+)-DIHYDROCARVONE INTO CHIRAL INTERMEDIATES FOR THE SYNTHESIS OF (-)-POLYGODIAL, (-)-WARBURGANAL AND (-)-MUZIGADIAL", TETRAHEDRON., Bd. 45, Nr. 5, 1989, Seiten 1447-1452, XP002690736, NLELSEVIER SCIENCE PUBLISHERS, AMSTERDAM. ISSN: 0040-4020

## Beschreibung

Die Erfindung betrifft eine Verfahren zur Herstellung von Drospirenon (6β,7β; 15β,16 β -dimethylene-3-oxo-17α-pregn-4-ene-21,17-carbolactone, **DRSP**) aus 6β,7β,15β,16β-dimethylen-5β-hydroxy-3-oxo-17α-androstan-21,17-carbolacton in isolierter Form durch Wasserabspaltung.

Drospirenon (6β,7β; 15β,16β-dimethylene-3-oxo-17α-pregn-4-ene-21,17-carbolactone, **DRSP,** INN) ist als steroidaler Wirkstoff seit längerem bekannt (DE 26 52 761 C2 und DE 30 22 337 A1) und die Herstellung der letzten 4 Schritte erfolgt im Eintopfverfahren; bei dem nach der Hydrierung von Dimethylenpropinol keine der durchlaufenen Zwischenstufen Dimethylenpropanol und 5-β-OH-DRSP isoliert werden (siehe nachfolgendes Schema).

Eine analoge Synthese, jedoch unter Anwendung einer Pyridiniumdichromat-Oxidation ist aus dem Stand der Technik bekannt [Angew. Chemie, 21, 9, (1982), Seiten 696 - 697]. Ähnliche Synthesen zur Herstellung von steroidalen 17, 21 - Carbolactonen werden auch innerhalb von EP-A-0 075 189 und EP-A-0 051 143 beschrieben, jedoch unter Beteiligung von mikrobiologischen Reaktionen. Oxidationen unter Beteiligung von Rutheniumverbindungen werden aber nicht darin offenbart.

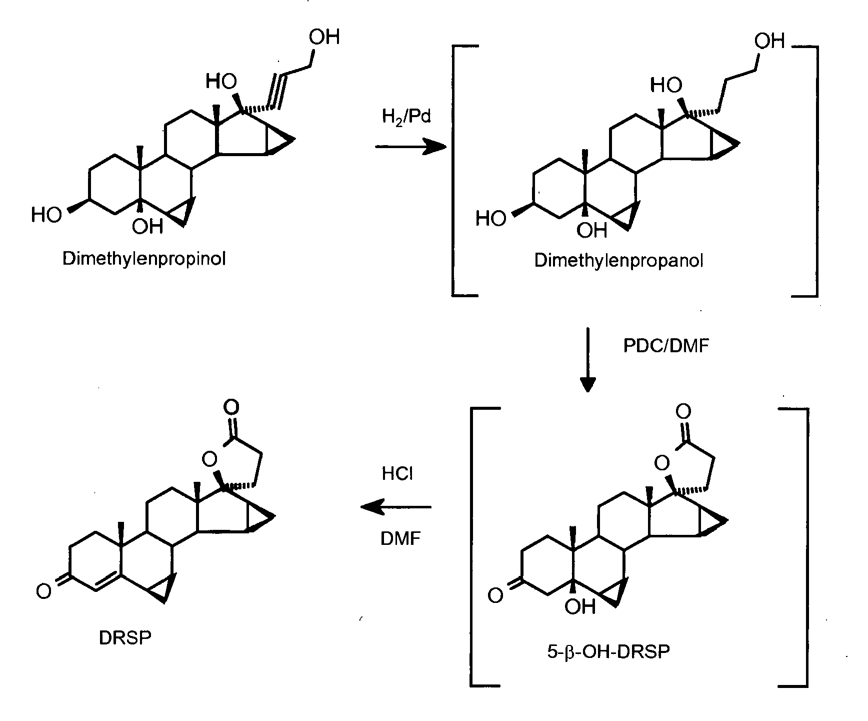

Das Dimethylenpropinol wird in THF mit Wasserstoff an Palladium-Kohle zum Dimethylenpropanol hydriert. Die so erhaltene Hydrierlösung, die das Propanol als Hauptprodukt und schwankende Anteile an Lactol enthält, wird ohne Isolierung und Zwischenaufarbeitung zum Drospirenon (DRSP) umgesetzt.

Hierzu wird zuerst ein Lösungsmittelwechsel von THF zu DMF vollzogen und anschließend das Propanol bei 40° C mit einem Überschuß von 3,7 Equivalenten Pyridiniumdichromat (PDC) zu einem Gemisch von DRSP und 5-β-OH-DRSP oxidiert. Die 5-β-OH-Funktion im Oxidationsprodukt ist labil gegenüber Säuren, Lewissäuren und basischen Bedingungen bei erhöhten Temperaturen, da in allen Fällen mit der Ausbildung des Δ-4,5-ungesättigten Ketons im Drospirenon ein thermodynamisch stabileres Produkt erhalten wird. Die Eliminierung der β-OH-Funktion im 5-β-OH-DRSP verläuft zum thermodynamisch stabileren Drospirenon und konnte nicht unterdrückt werden konnte.

Die Mischung enthält in der Regel wechselnde Anteile der beiden Komponenten, wobei das 5-β-OH-DRSP im allgemeinen als Hauptkomponente im Verhältnis von 2-3:1 vorliegt. In der letzten Stufe der Eintopfsequenz wird das Zweikomponenten-Gemisch durch Zugabe von halbkonzentrierter Salzsäure in das DRSP, roh überführt.

In der Nachstehenden Tabelle sind die letzten vier Betriebsansätze zusammengefaßt.

| Ansatz | Ausbeute, roh (%) | Reinheit (100%-Methode) |
|---|---|---|
| 1 | 57,2 | 98,9 |
| 2 | 63,7 | 99,09 |
| 3 | 46,5 | 99,18 |
| 4 | 58,3 | 98,81 |
| **Gesamt** | **mittlere Ausbeute: 56,4** | **mittlere Reinheit: 98,9** |

Im Mittel aller Betriebsansätze wird ausgehend vom Dimethylenpropinol eine theoretische Ausbeute von 56% DRSP, roh in einer HPLC-Reinheit von 98,9% erzielt.

Aufgabe der Erfindung ist die Bereitstellung eines neuen Herstellungsverfahrens für Drospirenon, welches selektiver und einfacher in der Durchführung ist, als jenes aus dem Stand der Technik und außerdem ökologischer ist; (Einsparung einer Chromtrioxid-Oxidation).

Gelöst wird diese Aufgabe gemäß der Lehre des Anspruchs. Die in der Stammanmeldung beschriebene Erfindung beinhaltet ein Verfahren zur Herstellung von Drospirenon (6β,7β; 15β,16β-dimethylene-3-oxo-17α-pregn-4-ene-21,17-carbolactone, **DRSP**) 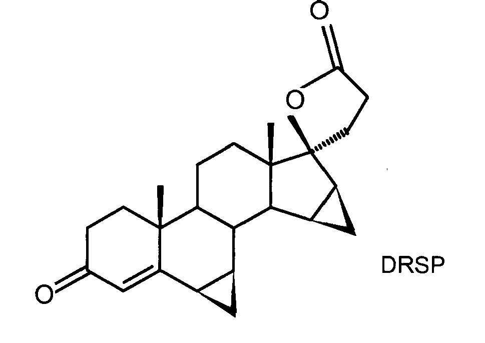 durch katalytische Hydrierung von 17α-(3-hydroxy-1-propynyl)-6β,7β;15β,16β-dimethylene-5β-androstane-3β,5,17β-triol 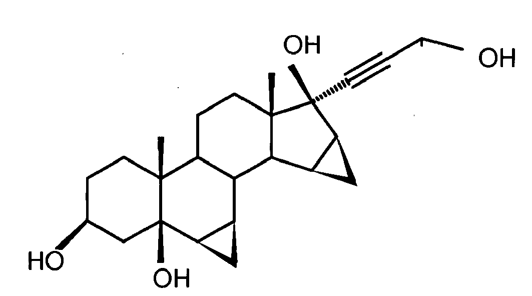 in das 7α-(3-hydroxy-1-propyl)-6β,7β; 15β,16β-dimethylene-5β-androstane-3β,5,17β-triol, 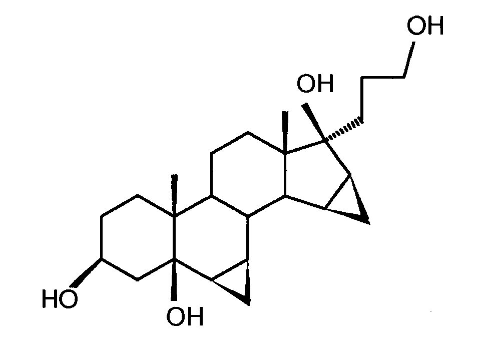 Oxidation in Gegenwart eines Rutheniumsalzes in das 6β,7β;15β,16β-dimethylene-5 β -hydroxy-3-oxo-17α-androstane-21,17-carbolactone 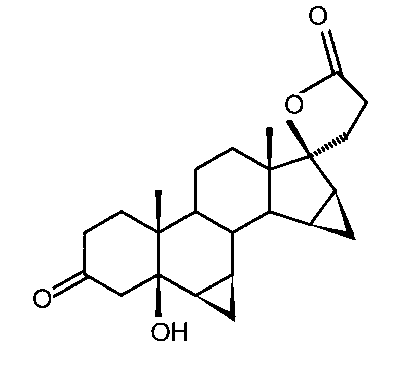 und anschließender Wasserabspaltung.

Die Erfindung beinhaltet als eine Schlüsselreaktion die Ruthenium katalysierte Oxidation von Dimethylenpropanol zum 5-β-OH-DRSP und die anschließende Wassereliminierung zum Drospirenon in einem zweistufigen Verfahren.

Analog zum bekannten Verfahren aus dem Stand der Technik wird im erfindungsgemäßen Verfahren Dimethylenpropinol in THF mit Wasserstoff an Palladium-Kohle hydriert. Die Hydrierlösung wird anschließend einem Lösungsmittelwechsel von THF auf Acetonitril unterworfen. Die Acetonitril-Lösung wird mit einer katalytischen Menge Rutheniumtrichlorid (1mol%) und 3 Equivalenten Natriumbromat bei 40°-60° C gezielt zum 5-β-OH-DRSP oxidiert.

Trotz der großen Labilität des 5-β-OH-DRSP gegenüber Säuren, Lewissäuren wie beispielsweise der Chromverbindungen im alten Betriebsverfahren, starken Basen oder hohen Temperaturen, die in allen Fällen auf die hohe Triebkraft zur Bildung des thermodynamisch stabileren Δ-4,5-ungesättigten Ketons zurückzuführen ist, gelingt unter den gewählten Reaktionsbedingungen die selektive Synthese des 5-β-OH-DRSP, ohne das eine Drospirenonbildung zu beobachten ist. Das 5-β-OH-DRSP kann durch eine (betrieblich) einfach durchzuführende Wasserfällung aus der Reaktionslösung isoliert werden.

Die Ausbeuten liegen im Bereich von 68% bis 75% über die beiden Stufen Hydrieren und anschließende Oxidation.

Aus eigenen Versuchen ist bekannt, daß das Drospirenon bei Säureeinwirkung nach zwei Reaktionswegen zersetzt werden kann. Einmal wird das Drospirenon unter sauren Bedingungen leicht in das epimere Isolacton überführt. 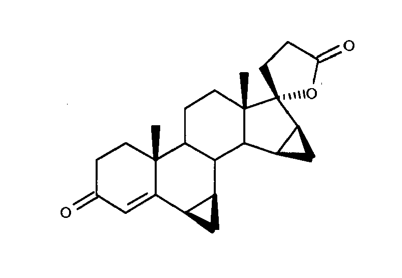 Das zweite Nebenprodukt entsteht durch einen HCl-Angriff auf die 6,7-Methylengruppe, der zu dem Ringöffnungsprodukt führt.

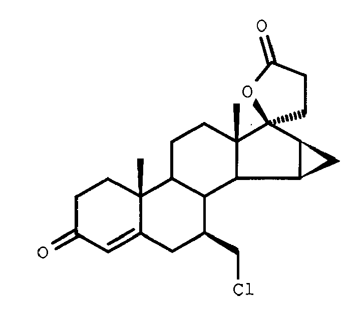

Beide Nebenprodukte, das Isolacton und das durch Säureeinwirkung gebildete Ringöffnungsprodukt, werden unter den Reaktionsbedingungen des neuen Verfahrens soweit zurückgedrängt, daß sie nur noch in einer Größenordnung von <0,2% zu beobachten sind.

Bei der Eliminierung wird eine Ausbeute von 96% d.Th. erzielt. Die Gesamtausbeute des neuen Verfahrens liegt damit im Bereich von 65% bis 72% d. Theorie.

Ein weiterer sehr wesentlicher Vorteil des erfindungsgemäßen Verfahrens gegenüber dem Stand der Technik liegt im Bereich der Ökologie. Es ist gelungen, die bisher verwendeten toxischen Chromverbindungen, die in Form der PDC-Salze bislang zur Oxidation verwendet wurden und hinterher in Form ihrer Lösungen entsorgt werden müssen, durch katalytische Mengen eines Metalls zu ersetzen. Zudem ist es möglich, daß eingesetzte Acetonitril-Wasser-Gemisch durch azeotrope Destillation zu recyclen, so daß auch keine Gefahr für die Umwelt zu erwarten ist.

### Beispiele

### 6β,7β;15β,16β-dimethylene-5β-hydroxy-3-oxo-17α-androstane-21,17-carbolactone

50 g 17α-(3-Hydroxy-1-propynyl)-6β,7β; 15β,16β-dimethylene-5β-androstane-3β,5,17 β -triol wird in 1000 ml THF in der Gegenwart von 10 g Palladium auf Kohle (10%) und 3 ml Pyridin hydriert bis 2 Equivalente Wasserstoff aufgenommen werden. Anschließend wird der Katalysator abfiltriert und die Lösung bis zur Trockne eingeengt, wobei 52,7 g 7α-(3-hydroxy-1-propyl)-6β,7β; 15β,16β-dimethylene-5β-androstane-3β ,5,17β-triol erhalten werden, die ohne Reinigung weiter umgesetzt werden.

50, 2 g 7α-(3-hydroxy-1-propyl)-6β,7β; 15β,16β-dimethylene-5β-androstane-3β,5,17β -triol werden in 250 ml Acetonitril suspendiert und auf 45° C erwärmt. Hierzu werden 0,52g Rutheniumtrichlorid gelöst in 10 ml Wasser und 62,46 g Natriumbromat gelöst in 250 ml Wasser getropft. Es wird 2 Stunden bei 50° C nachgerührt und die Lösung anschließend durch Zugabe von 1000 ml Wasser gequencht. Es werden 200 ml Ethylacetat zugesetzt, die Phasen getrennt und anschließend die wässrige Phase mit 600 ml Ethylacetat extrahiert. Die vereinigten organischen Phasen werden über Natriumsulfat getrocknet und anschließend bis zur Trockne eingeengt. Hierbei werden 43,44 g 6β,7β;15β,16β-Dimethylene-5β-hydroxy-3-oxo-17α-androstane-21,17-carbolactone als Rohprodukt erhalten. Durch Umkristallisieren aus Aceton-Isoether erhält man 35,7g 6β,7β;15β,16β-Dimethylene-5β-hydroxy-3-oxo-17α-androstane-21,17-carbolactone mit einem Schmelzpunkt von 216°-218°C. Der Drehwert liegt bei -65,6° (Natriumlinie, c=1,02 in CHCl3).

### 6β,7β; 15β,16β-Dimethylene-3-oxo-17α-pregn-4-ene-21,17-carbolactone

28 g 6β,7β;15β,16β-dimethylene-5β-hydroxy-3-oxo-17α-androstane-21,17-carbolactone werden in 280 ml THF suspendiert und anschließend mit 10 mol%, 1,5g p-Toluolsulfonsäure versetzt. Nach 30 Minuten werden 125 ml ges. NaCl-Lösung und 8,2 ml 1n NaOH-Lösung zugegeben. Nach Phasentrennung wird die organische Phase über Natriumsulfat getrocknet und bis zur Trockne eingeengt, wobei 25,67g 6 β ,7β; 15β,16β-dimethylene-3-oxo-17α-pregn-4-ene-21,17-carbolactone als Rohprodukt erhalten, dessen Reinheit nach HPLC-Bestimmung bei 93% liegt.

Zur weiteren Aufreinigung bietet sich entweder eine dreimalige Umkristallisation aus Methanol-Isoether an, die ein Produkt mit einer Reinheit > 99,5% nach HPLC liefert. Alternativ kann auch eine HPLC-Reinigung mit Hexan-Essigester=1:1 als Eluens an 10µm Kromasil durchgeführt werden, die ebenfalls 6β,7β; 15β,16β-Dimethylene-3-oxo-17α-pregn-4-ene-21,17-carbolactone mit einer Reinheit >99,5% liefert.

Der Schmelzpunkt der chromatographierten Substanz liegt bei 197,5° -200° C.

## Patentansprüche

1. Verfahren zur Herstellung von Drospirenon (6β,7β; 15β,16β-dimethylene-3- oxo-17α-pregn-4-ene-21,17-carbolactone, DRSP)
aus 6β,7β, 15β, 16β-dimethylen-5β-hydroxy-3-oxo-17α-androstan-21,17- carbolacton in isolierter Form durch Wasserabspaltung.

## Claims

1. Process for preparing drospirenone (6β,7β; 15β,16β-dimethylene-3-oxo-17α-pregn-4-ene-21,17-carbolactone, **DRSP**) from 6β,7β,15β,16β-dimethylene-5β-hydroxy-3-oxo-17α-androstane-21,17-carbolactone in isolated form by water elimination.

## Revendications

1. Procédé de préparation de la drospirénone (6β, 7β;
15β,16β-diméthylène-3-oxo-17α-prégn-4-ène-21,17-carbolactone, **DRSP**) à partir de la 6β, 7β, 15β, 16β-diméthylène-5β-hydroxy-3-oxo-17α-androstan-21,17-carbolactone en forme isolé par l'élimination d'eau.
